# EUROPEAN PATENT APPLICATION

(11) **EP 0 978 507 A1**
(43) Date of publication of application: **09.02.2000**
(21) Application number: 98202238.6
(22) Date of filing: 06.07.1998
(51) Int. Cl.: C07C 233/18, C08F 20/36

(54) **Radiation curable acrylic acid esters containing amide groups**

(71) Applicant: DSM N.V., 6411 TE Heerlen (NL)
(72) Inventor: Jansen, Johan Franz Gradus Antonius, 6165 AP Geleen (NL); Dias, Aylvin Jorge Angelo Athanasius, 6221 JD Maastricht (NL); Dorschu, Marko, 6411 CM Heerlen (NL)

(57) **Abstract**

The invention relates to a radiation curable compound such as (I) comprising a mono or multi valent carboxylic ester of a γ, δ or ε-hydroxyalkylamide group containing compound, in which the carboxylic ester is derived from an α,β-ethylenically unsaturated carboxylic acid.

A coating composition comprising this compound has a high rate of polymerisation and results in the desired chemical and mechanical properties.

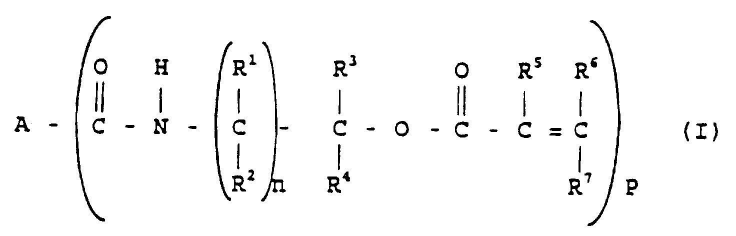

## Description

The invention relates to a radiation-curable compound and a composition comprising this compound.

During radiation curing processes the transformation of the fluid applied film to a solid crosslinked network can be considered to progress through three distinct stages being induction, polymerisation and attainment of maximum cure plateau. (Chemistry and Technology of UV and EB formulations, Volume IV, Oldring, 1991, pages 8-12).

Factors which improve or inhibit cure rate are, for example, the lamp system (UV-dose, intensity, wavelength, IR-content) and the chemical system (reactivity, absorption, coating weight, pigmentation, temperature, oxygen inhibition and substrate).

For commercial coating operations, it is necessary that the coating achieves a tackfree surface within seconds or less, because the interval between application of the coating and stacking or rewinding of the coated substrate is very short. Failure of the coating to achieve a non-tacky surface in this brief interval will result in the layers of coated substrate (in a stack or roll) sticking together ("blocking).

It is the object of the present invention to provide a coating composition having a high cure rate or rate of polymerisation and having the desired chemical and mechanical properies.

The radiation curable compound according to the invention is a mono or multi valent carboxylic ester of a γ , δ or ε-hydroxyalkylamide group containing compound, in which the carboxylic ester is derived from an α,β-ethylenically unsaturated carboxylic acid.

The composition based on the compound according to the invention results in high maximum polymerization rates.

According to a preferred embodiment of the invention the compound has formula (I): 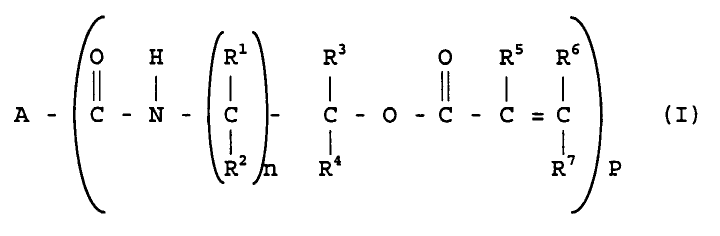 where:
- A =: hydrogen, or a monovalent or polyvalent organic group which is derived from a saturated or an unsaturated (C₁-C₆₀) alkyl group, or derived from an (C₆-C₁₀) aryl group,
- R¹, R², R³, R⁴: are, identical or different,
hydrogen or a lineair, branched or cyclic (C₁-C₈) alkyl chain,
- R⁵ =: hydrogen, (C₁-C₅)alkyl, -CH₂OH or CH₂COOX,
- R⁶,R⁷ =: hydrogen, (C₁-C₈) alkyl, (C₆-C₁₀)aryl or COOX,
- X =: hydrogen or (C₁-C₈) alkyl,
- n =: 2-4 and
- p =: 1-4

R¹, R² or R³ may form part of a cycloalkyl group.

The organic groups in A may furthermore be substituted with, for example, an ether, an ester, a hydroxy, an amide, an acid, an amine, an urethane, an urea, or a ketone.

Preferably, ester- or hydroxylgroups are applied as substitutents.

Preferably, A is a monovalent organic group which is derived from a saturated (C₁-C₁₂) alkyl group.

According to another preferred embodiment of the invention A is a polyvalent organic group derived from a saturated (C₂-C₁₀) alkyl group or a C₆-aryl group.

Preferably, R¹, R², R³ and R⁴ are hydrogen or methyl.

R⁵ is preferably hydrogen or (m)ethyl.

R⁶ and R⁷ are preferably hydrogen.

Preferably p is 1 or 2.

The compound can be obtained, for instance, by an esterification reaction between a γ, δ or ε-hydroxyalkylamide and an unsaturated carboxylic acid, at a temperature between, for example, 80°C and 140°C.

Preferably, 1 - 1.5 mol of acid are used per mole of hydroxide.

Preferably, the reaction takes place in the presence of an organic solvent, such as, for example, xylene, toluene or tetrahydrofuran.

Preferably, the reaction takes place in the presence of a stabilizing compound which prevents polymerization of the unsaturated ester groups under the conditions used for effecting this reaction. The stabilising compound or a mixture of stabilising compounds is generally used in amounts between about 50 and about 2000 ppm and preferably between 75 and 1000 ppm. They can be used in aerobic or anaerobic conditions depending on the stabilising compound.

Suitable stabilizing compounds include, for example, hydroquinone, monomethylhydroquinone, anthraquinone, β-nitrostyrene, phenothiazine and 2,6-di-tert-butyl-4-methyl-phenol (BHT).

The esterification reaction may take place in the presence of a catalyst. Suitable catalysts include strong acids, for example, sulphur-containing organic acids like alkane sulphonic acids and methane sulphonic acid.

Suitable unsaturated carboxylic acids include, for example, (meth)acrylic acid and derivatives, crotonic acid, (semi-ester of) itaconic acid, maleic acid, citaconic acid, mesaconic acid and fumaric acid.

Preferably, (meth)acrylic acid is used.

Suitable hydroxyalkylamides are, for example, N,N'-bis (γ-hydroxy propyl)-1,6-hexane diamide, N,N'-bis (δ-hydroxy butyl)-1,6-hexane diamide, N,N'-bis(1,6-hexane diamide, γ-hydroxy propyl acetamide, δ-hydroxy butyl acetamide and ε-hydroxy pentyl acetamide.

The compound according to the invention can also be obtained by the reaction between of a γ, δ or ε-hydroxyalkyl amide and an unsaturated carboxylic acid chloride, anhydride or ester.

The reaction between the amide and the unsaturated chloride or anhydride preferably takes place at temperature between 0°C and 30°C in a solvent in the presence of a base. Suitable solvents include, for example, tetrahydrofuran, dichloromethane and diethylether. Suitable bases include, for example, pyridine and triethylamine.

Suitable chlorides, anhydrides or esters include the chlorides, anhydrides and esters of the in the foregoing mentioned carboxylic acid.

The reaction between the amide and the unsaturated ester, preferably, takes place at temperatures between 80°C and 140°C in the presence of a Lewis acid.

Preferably, an excess of the unsaturated ester is applied. The ester functions both as solvent and as reactant.

Suitable Lewis acids are, for example, tetra alkyl titanate and sulphuric acid.

The compound applied in the invention can be cured by means of a free-radical reaction. In these reactions the free radicals can be obtained by radiation initiation.

Radiation-curing preferably takes place by means of, for example, a photochemical process such as, for example, ultraviolet radiation (UV) or a radiation-chemical process such as electron beam (EB).

UV and EB radiation are explained in greater detail by for example Bett et al. in the article entitled "UV and EB curing" (Jocca 1990 (11), pages 446 - 453).

The amount of the compound according to the invention can range between 0,01% by weight and 100% by weight in the radiation curable composition.

Generally, the radiation curable composition according to the invention is substantially solvent free.

The composition according to the invention can be used, for example, in coating compositions, inks and adhesives.

If desired and depending on the application, the compound can be combined with oligomers or polymers which are based, for example, on (meth)acrylate units, maleate units, fumarate units, itaconate units, vinylester units and/or vinylether units.

Due to the relatively high cure speeds the present compounds can also be applied as additives for enhancing the cure speed of a formulation. In general such additives are used in amounts ranging between 0,01% and 25% by weight and preferably in amounts between 0,5% and 10% by weight relatively to the total amount of all ingredients.

After curing the coatings according to the invention have many desired properties such as for example good chemical properties (resistance to solvents, acids, alkali and moisture), good optical properties and appearance, good mechanical properties (such as hardness, flexibility, adhesion, abrasion resistance, strength and durability), good thermal stability and good weatherability.

The composition comprising the radiation curable binder composition may further comprise pigments, stabilisers and other additives.

The radiation curable formulation generally consists of a prepolymer, a reactive diluent and additives. Two other possible components, depending upon the type of formulation and cure mechanism are pigments and photoinitiator system.

The compound according to the invention can be applied, for example, in a water based coating composition, in a solvent based coating composition, in a high solids coating composition, in a 100% solids coating composition and as a powder paint composition.

The most preferred irradiation source is ultraviolet light. Ultraviolet light is preferably high intensity light to provide a dosage to achieve reasonable curing rates. In the event that lower energy light is to be applied, it may then be desired to subject the compositions also to elevated temperatures in order to reduce the time for adequate polymerization to occur.

With respect to UV curing equipment we refer to, for example, pages 161-234 of Chemistry and Technology of UV and EB-formulations, Volume 1, Oldring, 1991.

Suitable lamps employed to provide the desired high intensity and availability of wavelength and spectral distribution include for example that available from Fusion Systems, Corp.

A composition according to the present invention can be applied on substrates such as, for example, plastic, paper, board, leather, glass, wood and metal.

This composition is preferably polymerised in the presence of a photoinitiator but it is also possible to polymerise in the absence of a photoinitiator.

Suitable photoinitiators allow for initiation of the curing process with exposure to light having wavelengths between about 200 nm and about 600 nm. Suitable photoinitiators have ketone functionalities and can be aromatic such as, for example, benzophenone. Darocur 1173® (Ciba) is a suitable benzyl-ketal-based photoinitiator, which contains 2-hydroxy-2-methyl-1-phenylpropane-1-one as an active component. Irgacure 184® (Ciba) is an aryl ketone containing hydroxycyclohexyl phenyl ketone as active component, and is a suitable photoinitiator. Irgacure 369® (active component 2-benzyl-2-dimethylaminol-1-(4-morpholinophenyl)-butanone-1) is also suitable. Acyl phosphines, such as for example 2,4,6,-trimethylbenzoyl diphenyl phosphone oxide (Lucerine TPO®, BASF) can also be used, as can Quantacure CPTX® (Octel Chemicals), which contains 1-chloro-4-propoxy thioxanthone as active component. Chemical derivatives of these photoinitiators are suitable, as are mixtures of these photoinitiators. A suitable combination of photoinitiators is Irgacure 1800™ (Ciba) consisting of 75% by weight Irgacure 184™ and 25% by weight (bis-(2,6-dimethoxy benzoyl)-2,4,4-trimethylpentyl fosfine oxide). Other suitable photoinitiators can be of the Norrish-II-type, for example, the combinations benzophenone with amine, maleimide with amine, thioxantone with amine and antrachinon with amine.

The invention is explained by reference to the following non-restrictive examples.

In the following the cure behaviour monitored with "real time infra red spectroscopy". The conversion of the double bonds during the photopolymerisation was monitored by means of infrared (Bruker IFS55).

### Example I

### Synthesis of γ-acryloyloxy-propyl propionamide

7.93 grams of acryloylchloride were slowly added to a stirred solution of 7.83 grams of γ-hydroxy-propyl propionamide and 5.08 grams of pyridine in 100 ml of dry tetrahydrofurane while maintaining the temperature below 5°C. After the addition the temperature was slowly raised to roomtemperature and the reaction mixture was stirred at this temperature for 18 hours.
The reaction mixture was filtered to remove the pyridine-HCl salt and subsequently evaporated followed by a bulb to bulb destilation to yield γ-acryloyloxy-propyl propionamide as an oil.

### Example II

### Synthesis of δ-acryloyloxy-butyl propionamide

7.93 grams of acrylcylchloride were slowly added to a stirred solution of 8.48 grams of δ-hydroxy-butyl propionamide and 5.08 grams of pyridine in 100 ml of dry tetrahydrofuran while maintaining the temperature below 5°C. After the addition the temperature was slowly raised to roomtemperature and the reaction mixture was stirred at this temperature for 18 hours. The reaction mixture was filtered to remove the pyridine-HCl salt and subsequently evaporated followed by a bulb to bulb distilation to yield δ-acryloyloxy-butyl propionamide as an oil.

### Example III

### Synthesis of ε-acryloyloxy-pentyl propionamide

7.93 grams of acryloylchloride were slowly added to a stirred solution of 9.29 grams of ε-hydroxy-pentyl propionamide and 5.08 grams of pyridine in 100 ml of dry tetrahydrofuran while maintaining the temperature below 5°C. After the addition the temperature was slowly raised to roomtemperature and the reactionmixture was stirred at this temperature for 18 hours.
The reaction mixture was filtered to remove the pyridine-HCl salt and subsequently evaporated followed by a bulb to bulb distilation to yield ε-acryloyloxy-pentyl propionamide as an oil.

### Comparative Example A

### Synthesis of ζ-acryloyloxy-hexyl propionamide

To a stirred solution of 10.11 grams of ζ-hydroxy-hexyl propionamide, 5.08 grams of pyridine in 100 ml of dry tetrahydrofuran were slowly added 7.93 grams of acryloylchloride maintaining the temperature below 5°C. After the addition the temperature was slowly raised to roomtemperature and the reaction mixture was stirred at this temperature for 18 hours. The reaction mixture was filtered to remove the pyridine-HCl salt and subsequently evaporated followed by a bulb to bulb distilation to yield ζ-acryloyloxy-hexyl propionamide as an oil.

### Example IV

### Curing of γ-acryloyloxy-propyl propionamide

20 mg Irgacure 184™ were dissolved in 2 grams of γ-acryloyloxy-propyl propionamide according to Example I. A 10µm thick film was applied on a gold coated alumina plate and the film was cured in an infrared spectrophotometer (Bruker IFS-55) equipped with a 400W mercury halide lamp. Due to this set up the conversion of acrylate bonds of γ-acryloyloxy-propyl propionamide can be monitored in situ during irradiation. The results are shown in table I.

### Example V

### Curing of δ-acryloyloxy-butyl propionamide

20 mg Irgacure 184™ were dissolved in 2 grams of γ-acryloyloxy-butyl propionamide according to Example II. A 10µm thick film was applied on a gold coated alumina plate and the film was cured in an infrared spectrophotometer (Bruker IFS-55) equipped with a 400W mercury halide lamp. Due to this set up the conversion of acrylate bonds of γ-acryloyloxy-butyl propionamide can be monitored in situ during irradiation. The results are shown in Table I .

### Example VI

### Curing of ε-acryloyloxy-pentyl propionamide

20 mg Irgacure 184™ were dissolved in 2 grams of ε-acryloyloxy-pentyl propionamide according to Example III. A 10µm thick film was applied on a gold coated alumina plate and the film was cured in an infrared spectrophotometer (Bruker IFS-55) equipped with a 400W mercury halide lamp. Due to this set up the conversion of acrylate bonds of ε-acryloyloxy-pentyl propionamide can be monitored in situ during irradiation. The results are shown in table I.

### Comparative Example B

### Curing of ζ-acryloyloxy-hexyl propionamide

20 mg Irgacure 184™ were dissolved in 2 grams of ζ-acryloyloxy-hexyl propionamide (according to Comparative Example A). A 10µm thick film was applied on a gold coated alumina plate and the film was cured in an infrared spectrophotometer (Bruker IFS-55) equipped with a 400W mercury halide lamp. Due to this set up the conversion of acrylate bonds of ζ-acryloyloxy-hexyl propionamide can be monitored in situ during irradiation. The results are shown in Table I.

### Comparative Example C

### Curing of lauryl acrylate

20 mg Irgacure 184™ were dissolved in 2 grams of lauryl acrylate. An 10µm thick film was applied on a gold coated alumina plate and the film was cured in an infrared spectrophotometer (Bruker IFS-55) equipped with a 400W mercury halide lamp. Due to this set up the conversion of acrylate bonds of lauryl acrylate can be monitored in situ during irradiation. The results are summarized in Table I.

**Table I**

| Example | rate of double bond conversion (%/sec) | % conversion after 2 sec |
|---|---|---|
| IV | 123 | 97 |
| V | 116 | 97 |
| VI | 108 | 96 |
| Comp. B | 35 | 70 |
| Comp. C | 22 | 47 |

The compounds according to the invention can be applied as monomers in radiation curable compositions and they result in a high cure rate.

## Claims

1. A radiation curable compound being a mono or multi valent carboxylic ester of a γ, δ or ε-hydroxyalkylamide group containing compound, in which the carboxylic ester is derived from an α,β-ethylenically unsaturated carboxylic acid.

2. Compound according to Claim 1, characterized in that the compound is according to formula (I): 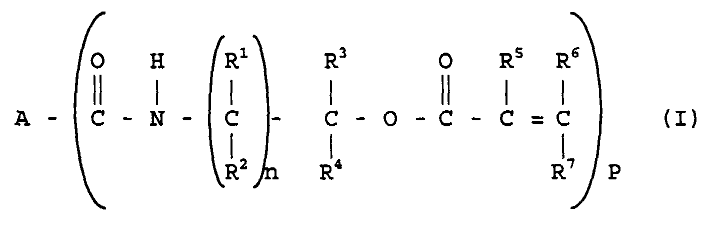 where:
A = hydrogen, or a monovalent or polyvalent organic group which is derived from a saturated or an unsaturated (C₁-C₆₀) alkyl group, or derived from an (C₆-C₁₀) aryl group,
R¹, R², R³, R⁴ are, identical or different,
hydrogen or a lineair, branched or cyclic (C₁-C₈) alkyl chain,
R⁵ = hydrogen, (C₁-C₅)alkyl, -CH₂OH or CH₂COOX,
R⁶,R⁷ = hydrogen, (C₁-C₈) alkyl, (C₆-C₁₀)aryl or COOX,
X = hydrogen or (C₁-C₈) alkyl,
n = 2-4 and
p = 1-4

3. Compound according to Claim 2, characterized in that A is a monovalent organic group derived from a saturated (C₁-C₁₂) alkyl group or A is a polyvalent organic group derived from a saturated (C₂-C₁₀) alkyl group or a C₆-aryl group.

4. Compound according to any one of Claims 2-3, characterized in that R¹, R², R³ and R⁴ are hydrogen or methyl, R⁵ is hydrogen or (m)ethyl and R⁶ and R⁷ are hydrogen.

5. A radiation curable composition comprising a compound according to any one of Claims 1-4.

6. A radiation curable coating composition comprising a compound according to any one of Claims 1-4.

7. A coating obtained by radiation curing of a composition according to Claim 5.

8. Entirely or partly coated substrate wherein a coating according to Claim 7 is applied as the coating.
